# FASCICULE DE BREVET EUROPEEN

(11) **EP 4 440 668 B1**
(45) Date de publication et mention de la délivrance du brevet: **03.06.2026**
(21) Numéro de dépôt: 22834686.2
(22) Date de dépôt: 30.11.2022
(51) Int. Cl.: A61M 16/06, A61M 11/00

(54) **SYSTÈME D'INHALATION COMPRENANT UN NÉBULISEUR À TAMIS ET UN MASQUE FACIAL**
INHALATIONSSYSTEM MIT EINEM MESH-VERNEBLER UND EINER GESICHTSMASKE
INHALATION SYSTEM COMPRISING A MESH NEBULIZER AND A FACIAL MASK

(30) Priorité: 01.12.2021 FR 2112823
(43) Date de publication de la demande: 09.10.2024
(73) Titulaire: Université de Tours, 37020 Tours Cedex 1 (FR); INSERM (Institut National de la Santé et de la Recherche Médicale), 75013 Paris (FR)
(72) Inventeur: VECELLIO, Laurent, 37170 Chambray les Tours (FR)
(74) Mandataire: Icosa
(86) Numéro de dépôt international: PCT/FR2022/052203
(87) Numéro de publication internationale: WO 2023/099842

(56) Documents cités:
- WO-A1-2017/131607
- CN-A- 113 181 496
- CN-U- 202 409 763
- CN-U- 204 428 562
- CN-U- 206 675 800
- CN-U- 211 357 213
- CN-U- 214 912 223
- FR-A1- 2 886 127
- US-A1- 2003 024 533
- US-A1- 2012 172 740

## Description

### DOMAINE DE L'INVENTION

La présente invention concerne un système d'inhalation et son utilisation pour administrer un principe actif dans les voies respiratoires d'un utilisateur.

Plus précisément, la présente invention concerne un système d'inhalation d'un aérosol produit par un nébuliseur à tamis.

### ÉTAT DE LA TECHNIQUE

L'aérosolthérapie consiste en l'administration d'un aérosol dans les voies respiratoires (ou voies aériennes) d'un utilisateur. Cette technique peut être utilisée à différentes fins comme, par exemple, l'administration de principes actifs qui vont agir localement sur les voies respiratoires, l'humidification les voies respiratoires pour fluidifier les sécrétions bronchiques ou encore l'administration d'un pathogène ou d'un marqueur dans le cadre de tests cliniques ou de recherche. L'aérosol est produit par un nébuliseur qui transforme le liquide en fines gouttelettes (aérosol) qui sont injectées dans un masque (nasal, facial, buccal ou narinaire) afin d'être respirées par l'utilisateur.

Cependant, les systèmes d'inhalation connus ne sont pas satisfaisants.

En effet, le dépôt de l'aérosol dans les poumons de l'utilisateur est peu efficace lors de l'utilisation des systèmes connus car, durant la phase expiratoire, une proportion non négligeable de gouttelettes est perdue dans l'air ambiant. De plus, le volume du masque comprend un volume mort limitant la pénétration de l'aérosol dans les voies respiratoires de l'utilisateur ce qui implique un dépôt de l'aérosol principalement dans les voies respiratoires supérieures et, pour une faible partie, dans les poumons. Cet effet est particulièrement remarqué chez les jeunes enfants qui possèdent un volume d'inspiration beaucoup plus faible que les adultes et dans certains cas, plus faible que le volume du masque.

Cette faible efficacité du dépôt de l'aérosol dans les poumons peut aussi être causée par le type de nébuliseur utilisé. En effet, la plupart des nébuliseurs connus possèdent un rendement faible impliquant la présence d'une forte proportion (jusqu'à 50%) de liquide résiduel dans le nébuliseur en fin de séance.

Certains systèmes sont munis d'un nébuliseur à tamis connecté sur une pièce en « T » munie de deux ouvertures dont l'une d'elle est connectée à un masque et l'autre est laissée libre à l'air ambiant. Ainsi, en phase expiratoire, l'air expiré par le patient sort par l'ouverture laissée libre et entraine l'aérosol produit par le nébuliseur vers l'air extérieur générant ainsi des pertes d'aérosols. Durant la phase inspiratoire, l'air extérieur entre par l'ouverture laissée libre et entraine l'aérosol produit par le nébuliseur vers le masque jusqu'au patient. L'intérieur du masque étant dépourvu d'aérosol durant le début de l'inhalation, une partie de l'air inspiré ne contient pas d'aérosol. Cela réduit donc la quantité d'aérosol qui pénètre dans les voies respiratoires. La quantité d'aérosol qui pénètre dans les voies respiratoires est réduite dans les masques comprenant un nébuliseur débouchant directement dans la partie centrale du masque et injectant l'aérosol de manière perpendiculaire au niveau du visage de l'utilisateur. Néanmoins, durant la pause respiratoire ou lors de faibles débits inspiratoires, lorsque l'utilisateur est assis ou couché, l'aérosol est directement projeté et déposé sur la face de l'individu. De plus, durant la phase expiratoire la direction de l'aérosol est modifiée par le flux d'air expiré par le patient. Le flux d'air situé en face de la production d'aérosol l'entraine vers l'orifice de sortie du masque créant une perte d'aérosol durant la phase expiratoire.

La demande de brevet chinoise CN 113 181 496 divulgue un système d'inhalation comprenant un nébuliseur à tamis et un masque facial. Cependant, l'introduction de l'aérosol dans le masque n'est pas dirigée vers la partie nasale.

Le modèle d'utilité chinois CN 206 675 800 divulgue un système d'inhalation comprenant un nébuliseur et un masque facial. Cependant, l'introduction de l'aérosol dans le masque n'est pas dirigée vers la partie nasale.

La demande de brevet français FR 2 886 127 divulgue un système d'inhalation comprenant un nébuliseur et un masque facial. Cependant, les flux entrant et sortant lors de la respiration du patient auquel est appliqué le masque ne sont pas dirigés vers la partie nasale (pour l'aérosol introduit dans le masque) ou de la partie buccale vers l'extérieur (pour l'expiration).

Enfin, le modèle d'utilité chinois CN 214 912 223 divulgue un masque d'atomisation pour enfant permettant d'administrer un médicament. Toutefois, les flux entrant et sortant lors de la respiration du patient auquel est appliqué le masque ne sont pas clairement décrits.

De plus, certains systèmes, tels que ceux décrits dans les demandes de brevets français FR2985909 et FR2879465, sont équipés de valves - éventuellement unidirectionnelles - permettant l'entrée d'air ou d'aérosol dans le masque par une ouverture différente de la sortie d'air. Cependant, l'ouverture de ces valves n'est pas déclenchée instantanément au début de chaque phase inspiratoire à cause de la propriété de détente des gaz dans le masque, en particulier pour les petits volumes respiratoires. Cet effet contribue à une diminution de l'administration de l'aérosol dans le masque et donc aussi à une réduction de l'efficacité de ces systèmes. Par ailleurs, dans ce type de système, le volume du masque représente toujours un espace mort réduisant la quantité d'aérosol inhalée par le patient.

En outre, les systèmes connus conduisent au dépôt de l'aérosol sur les parois internes du masque à cause de l'effet de coalescence qui provoque l'agglomération des gouttelettes entre elles et de l'effet de condensation. Les gouttelettes de l'aérosol ainsi déposées et perdues ne contribueront donc pas au dépôt de l'aérosol dans les poumons.

Finalement, l'étanchéité des systèmes connus n'est pas optimale impliquant une expulsion de l'aérosol en dehors du masque lors de l'expiration ce qui entraîne un risque de contamination de l'air ambiant.

Le but de l'invention est de fournir un système qui permet d'éviter un ou plusieurs de ces problèmes en favorisant l'inhalation d'aérosol avec une plus grande efficacité de dépôt dans les voies respiratoires inférieures (les poumons) ou en diminuant la contamination de l'air extérieur ou le dépôt sur les parois du masque.

### RÉSUMÉ

Dans ce but, la présente invention concerne un système d'inhalation comprenant un nébuliseur à tamis et un masque facial comprenant :
- Un corps de masque configuré pour couvrir le nez et la bouche d'un utilisateur lorsque ledit corps de masque est placé sur l'utilisateur et délimité par un bord de masque définissant une surface caractérisée par un plan moyen,
- Une première ouverture comprenant un tube débouchant dans la partie nasale du corps de masque et comprenant une première extrémité dont l'intersection avec le corps de masque définit une surface dont la direction normale forme un angle supérieur à 10° et inférieur à 90°, de préférence inférieur ou égal à 75° avec le plan moyen et une deuxième extrémité connectée au nébuliseur à tamis, la partie nasale du corps de masque étant la partie du corps de masque située, lorsque le masque est porté par un utilisateur assis ou debout, au-dessus de la pointe du nez, et
- Une deuxième ouverture comprenant un tube débouchant dans la partie buccale du corps de masque et comprenant une première extrémité dont l'intersection avec le corps de masque définit une surface dont la direction normale forme un angle supérieur à 0° et inférieur à 90° avec le plan moyen, la partie buccale du corps de masque étant la partie du corps de masque située, lorsque le masque est porté par un utilisateur assis ou debout, en dessous de la lèvre inférieure.

En effet, l'utilisation d'un nébuliseur à tamis permet d'obtenir, grâce à l'absence d'injection de gaz dans l'aérosol, un rendement supérieur aux autres nébuliseurs connus entraînant ainsi une augmentation de l'efficacité de dépôt dans les voies respiratoires (ou voies aériennes) inférieures (poumons).

De plus, le masque facial couvrant le nez et la bouche de l'utilisateur, cela permet un traitement simultané des voies respiratoires supérieures et inférieures car l'utilisateur peut inhaler à la fois par le nez et la bouche. De plus, le nébuliseur étant connecté à la première ouverture débouchant dans la partie nasale (ou partie haute) du corps de masque, cela permet de diffuser l'aérosol de part et d'autre du nez de l'utilisateur et de le diriger, par gravité, vers la partie buccale du corps de masque durant l'expiration et la pause respiratoire. La deuxième ouverture débouchant dans la partie buccale (ou partie basse) du corps de masque, cela permet une augmentation de l'efficacité de dépôt dans les voies respiratoires par l'inhalation d'une plus grande proportion de l'aérosol qui s'est accumulé dans cette partie buccale du masque facial durant la phase expiratoire.

Selon un autre aspect avantageux de l'invention, la deuxième ouverture comprenant un tube comprend une seconde extrémité connectée à une valve unidirectionnelle permettant une communication de l'extérieur vers l'intérieur du corps de masque, le masque facial comprenant en outre une troisième ouverture comprenant un tube débouchant dans la partie centrale du corps de masque et comprenant une première extrémité dont l'intersection avec le corps de masque définit une surface dont la direction normale est sensiblement perpendiculaire au plan moyen.

En effet, l'inclusion d'une troisième ouverture débouchant dans la partie centrale du corps de masque permet de limiter l'expulsion de l'aérosol hors du corps de masque et donc la perte d'aérosol car, grâce à la diffusion de l'aérosol de part et d'autre du nez de l'utilisateur, l'aérosol est en dehors de la trajectoire de l'air expiré.

Cet avantage est d'autant plus marqué grâce à l'utilisation d'une valve unidirectionnelle dans la deuxième ouverture permettant ainsi de diriger l'air expiré uniquement à travers la troisième ouverture.

Selon un autre aspect avantageux de l'invention, la troisième ouverture comprenant un tube comprend une deuxième extrémité connectée à une valve unidirectionnelle permettant une communication de l'intérieur du corps de masque vers l'extérieur.

En effet, l'utilisation d'une valve unidirectionnelle dans la troisième ouverture permet l'inspiration de l'air extérieur uniquement à partir de la deuxième ouverture. Ceci permet une augmentation de l'efficacité de dépôt dans les voies respiratoires par l'inhalation de l'air comprenant la plus grande proportion d'aérosol, c'est-à-dire l'air contenu dans la partie buccale du masque facial.

Selon un autre aspect avantageux de l'invention, la dimension longitudinale des tubes de chacune des ouvertures est inférieure à 2 centimètres, de préférence inférieure à 1 centimètre.

En effet, une utilisation de tubes de petite taille permet d'orienter les flux d'air pénétrant dans le masque. De plus, grâce à la diminution du volume total du masque, cela permet un déclenchement plus rapide des valves unidirectionnelles qui peuvent être présentes dans les tubes du masque facial.

Selon un autre aspect avantageux de l'invention, la troisième ouverture comprend une deuxième extrémité connectée à un filtre.

En effet, l'utilisation d'un filtre permet de diminuer d'autant plus l'expulsion des gouttelettes d'aérosol hors du corps de masque et donc la contamination de l'air extérieur.

Selon un autre aspect avantageux de l'invention, la deuxième ouverture comprend en outre une entrée destinée à être reliée à une source de gaz sec, la valve unidirectionnelle de la deuxième ouverture étant située entre ladite entrée et le corps de masque.

En effet, l'utilisation d'une source de gaz sec connectée à la deuxième ouverture permet, durant la phase inspiratoire, d'aspirer de l'air sec ou de l'oxygène sec qui a pour effet de limiter la condensation de vapeur d'eau sur les gouttelettes évitant le dépôt à la surface interne du masque facial. Cela contribue à récupérer des gouttelettes d'aérosol et donc d'augmenter l'efficacité de dépôt dans les voies respiratoires.

Selon un autre aspect avantageux de l'invention, le corps de masque comprend en outre un répartiteur permettant de diriger un aérosol produit par le nébuliseur à tamis de part et d'autre du nez de l'utilisateur lorsque le masque facial est placé sur l'utilisateur, ledit répartiteur étant positionné entre la première ouverture et la troisième ouverture.

En effet, l'utilisation d'un répartiteur permet d'orienter le flux de l'aérosol de part et d'autre du nez tout en diminuant la quantité d'aérosol déposé sur le nez du patient. Cela contribue à la diminution de la contamination de l'air extérieur et à l'augmentation de l'efficacité de dépôt dans les voies respiratoires.

Selon un autre aspect avantageux de l'invention, le corps de masque possède un volume inférieur à 500 millilitres.

En effet, ce volume d'air correspond au volume moyen d'air inhalé par un adulte à chaque inspiration. Cela permet de réduire le volume mort et donc d'augmenter la proportion d'aérosol inhalé.

Selon un autre aspect avantageux de l'invention, le corps de masque possède un volume inférieur à 100 millilitres pour une utilisation chez un enfant.

En effet, ce volume d'air correspond au volume moyen d'air inhalé par un enfant à chaque inspiration. Cela permet de réduire le volume mort et donc d'augmenter la proportion d'aérosol inhalé.

Selon un autre aspect avantageux de l'invention, le bord du corps de masque est fabriqué dans un matériau souple.

En effet, cette caractéristique permet d'augmenter l'étanchéité du masque et ainsi de réduire la contamination de l'air extérieur.

La présente divulgation concerne aussi une utilisation du système selon un des modes de réalisation pour administrer un produit dans les voies respiratoires d'un utilisateur comprenant les étapes de :
- Introduction d'un principe actif en liquide dans le nébuliseur à tamis,
- Installation du système sur le nez et la bouche de l'utilisateur,
- Génération en continu d'un aérosol par le nébuliseur à tamis, de préférence avec un débit compris entre 0,01 et 1 millilitre/minute, de préférence entre 0,05 et 0,1 millilitre/minute et/ou une taille des gouttelettes de liquides de l'aérosol comprise entre 0,1 et 10 microns, de préférence comprise entre 2 et 5 microns.

En effet, l'utilisation de cette gamme de débit permet de limiter la perte d'aérosol par condensation des gouttelettes sur la surface interne du masque.

De plus, une taille de gouttelettes faible permet une meilleure efficacité de dépôt dans les voies respiratoires inférieures.

### DÉFINITIONS

Dans la présente invention, les termes ci-dessous sont définis de la manière suivante :
**"MMAD"** ou **"Median Mass Aerodynamic Diameter"** ou **"Diamètre Aérodynamique Massique Médian"** concerne la valeur médiane du diamètre des gouttelettes d'aérosol produit par le nébuliseur. Cette taille peut être mesurée, par exemple, par impacteur en cascade.

**"Partie buccale d'un masque"** concerne la partie d'un masque facial située, lorsque le masque est porté par un utilisateur assis ou debout, en dessous de la lèvre inférieure.

**"Partie centrale d'un masque"** concerne la partie d'un masque facial comprise, lorsque le masque est porté par un utilisateur, entre la lèvre inférieure et la pointe du nez.

**"Partie nasale d'un masque"** concerne la partie d'un masque facial située, lorsque le masque est porté par un utilisateur assis ou debout, au-dessus de la pointe du nez.

**"Rendement de nébulisation"** concerne le rapport entre la quantité d'aérosol déposée dans les voies respiratoires (inférieures et supérieures) sous forme de liquide en fin de séance de nébulisation et la quantité de liquide introduite dans le nébuliseur avant le début de la séance de nébulisation.

**"Rendement pulmonaire"** concerne le rapport entre la quantité d'aérosol déposée dans les voies respiratoires inférieures (les poumons) en fin de séance de nébulisation et la quantité de liquide introduite dans le nébuliseur avant le début de la séance de nébulisation.

**"Volume d'un masque"** concerne la somme du volume du corps de masque complété par une surface moyenne définie par le bord de masque et du volume des ouvertures complétées chacune par une surface moyenne définie par le bord de son extrémité ne débouchant pas dans le corps de masque.

**"Volume mort"** concerne le volume d'air contenu dans le masque facial et dépourvu d'aérosol inhalé à chaque inspiration.

### BRÈVE DESCRIPTION DES FIGURES

D'autres caractéristiques et avantages de la présente invention ressortiront de la description faite ci-dessous, en référence aux dessins annexés qui en illustrent des exemples de réalisation dépourvus de tout caractère limitatif. Sur les figures :
**Figure 1** montre un système d'inhalation selon un mode de réalisation de l'invention.
**Figure 2** montre le masque facial selon un mode de réalisation de l'invention tel qu'il est positionné durant son utilisation.
**Figure 3** montre le système d'inhalation dans le mode de réalisation dans lequel le masque facial comprend trois ouvertures.
**Figure 4** montre le système d'inhalation dans le mode de réalisation dans lequel le masque facial comprend trois ouvertures et deux valves unidirectionnelles.
**Figure 5** montre le système d'inhalation dans le mode de réalisation dans lequel le masque facial comprend trois ouvertures et un filtre.
**Figure 6** montre le système d'inhalation dans le mode de réalisation dans lequel le masque facial comprend trois ouvertures, deux valves unidirectionnelles et une entrée destinée à être reliée à un réservoir d'air sec.
**Figure 7** montre le masque facial dans le mode de réalisation dans lequel il comprend trois ouvertures et un répartiteur.
**Figure 8** montre l'utilisation du système d'inhalation selon l'invention durant un cycle respiratoire.
**Figure 9** montre une comparaison de l'efficacité du dépôt de l'aérosol dans les voies respiratoires en utilisant un masque facial connu de l'art antérieur (à gauche) et le masque facial selon un mode de réalisation de l'invention (à droite).
**Figure 10A** montre un flux d'aérosol généré dans le masque facial selon un mode de réalisation de l'invention tandis que la **Figure 10B** montre un flux d'aérosol généré dans un masque facial connu de l'art antérieur.
**Figure 11** montre une comparaison d'un masque facial connu de l'art antérieur (à gauche) et du masque facial selon un mode de réalisation de l'invention (à droite).

### DESCRIPTION DÉTAILLÉE

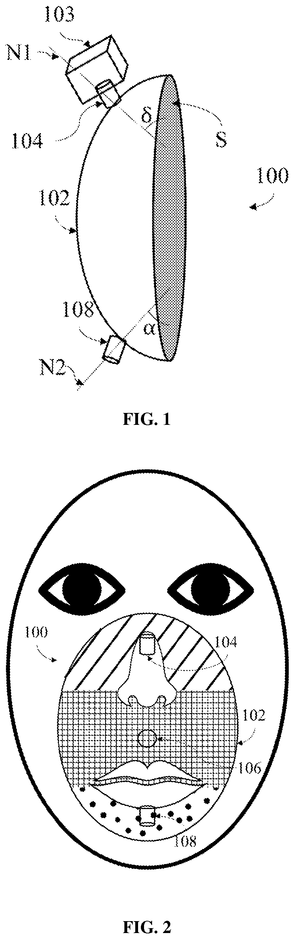

La figure 1 montre un système (100) d'inhalation comprenant notamment un masque facial. Le masque facial comprend un corps de masque (102), une première ouverture et une deuxième ouverture.

Le corps de masque (102) est configuré pour couvrir le nez et la bouche d'un utilisateur lorsque le système est placé sur (ou porté par) un utilisateur. Le port du masque permet de séparer virtuellement le corps de masque (102) en trois parties qui sont définies en fonction de la partie du visage couverte par chacune d'elles. Ainsi, comme représenté à la figure 2, le corps de masque (102) comprend une partie nasale (hachurée sur la figure 2) s'étendant, lorsque le masque est porté par un utilisateur, au-dessus la pointe du nez, une partie buccale (pointillée sur la figure 2) s'étendant en dessous de la lèvre inférieure et une partie centrale (en damier sur la figure 2) comprise entre la lèvre inférieure et la pointe du nez. De manière équivalente, ces parties peuvent respectivement correspondre au haut, au bas et au centre du masque, le haut et le bas étant définis verticalement lorsque l'utilisateur portant le masque est assis ou debout.

Le corps de masque (102) est délimité par un bord de masque qui peut être défini comme la partie du corps de masque (102) entrant en contact avec le visage d'un utilisateur lorsqu'il est porté. De manière avantageuse, le bord de masque peut être un élément ajouté au corps de masque (102) et fabriqué à partir d'un matériau souple. Par exemple, le matériau souple peut être un silicone ou autre élastomère souple, ou encore bourrelet gonflable permettant de s'adapter à la forme du visage. Ceci permet d'augmenter l'étanchéité du masque et donc de diminuer la contamination de l'air extérieur par l'aérosol et d'augmenter l'efficacité de dépôt de l'aérosol dans les voies respiratoires de l'utilisateur.

Le bord de masque définit une surface (en gris sur la figure 1) caractérisée par un plan moyen (S). Ainsi, le corps de masque (102) et le plan moyen (S) définissent une partie intérieure (ou interne) et une partie extérieure (ou externe) au masque facial. De plus, le corps de masque (102) peut aussi être caractérisé par un plan vertical, sensiblement perpendiculaire au plan moyen (S) et passant par l'axe de symétrie du visage de l'utilisateur.

La première ouverture comprend un tube (104), aussi appelé tube de nébulisation, débouchant dans la partie nasale (ou la partie haute) du corps de masque (102). Le tube (104) possède, par définition, deux extrémités. L'extrémité débouchant dans le corps de masque (102) forme, avec ce dernier, une surface d'intersection qui est caractérisée par une direction normale (N1). La direction normale (N1) forme un angle (δ) supérieur à 10° et inférieur ou égal à 90° avec le plan moyen (S), de préférence entre 10° et 75°. Avoir un angle (δ) inférieur à 75° est avantageux car cela permet de diriger le flux d'aérosol dans une direction longitudinale à la face du patient et vers le bas du masque. Ainsi, l'aérosol peut rapidement s'accumuler dans la partie basse du masque grâce à la vitesse initiale des particules et la gravité. La deuxième extrémité du tube (104) est connectée à un nébuliseur à tamis (103). Ainsi, le nébuliseur à tamis est connecté à l'extérieur du masque, ce qui permet d'intervenir sur le nébuliseur à tamis pendant l'utilisation du système d'inhalation. De plus, cette connexion à l'extérieur permet de maintenir une forme compacte pour le corps de masque, de sorte à en limiter son volume.

Un nébuliseur (ou brumisateur) permet de transformer les liquides en un nuage de gouttelettes extrêmement fines (aérosol). Le fonctionnement des nébuliseurs à tamis est basé sur le passage du liquide au travers des trous du tamis pour générer des gouttelettes d'un diamètre sensiblement équivalent aux diamètres des trous du tamis. Les nébuliseurs à tamis peuvent être à membrane statique ou vibrante. Dans le cas d'un nébuliseur à tamis à membrane vibrante, la force mécanique pour assurer le passage du liquide au travers du tamis peut être réalisée à l'aide d'un quartz piézo-électrique mis en vibration. Le quartz piézo-électrique peut également être associé au tamis. Dans ce cas, la vibration permet de créer un effet d'extrusion et de projeter le liquide au travers des trous produisant ainsi un aérosol calibré. L'utilisation d'un nébuliseur à tamis évite l'injection de gaz dans l'aérosol, et permet un rendement supérieur aux autres nébuliseurs connus entraînant ainsi une augmentation de l'efficacité de dépôt dans les voies respiratoires. Ce nébuliseur à tamis (103) est compris dans le système (100) selon la présente invention.

De manière avantageuse, l'axe longitudinal du tube de nébulisation (104) peut être compris dans le plan vertical du corps de masque (102). Ceci est avantageux car le tube (104) est alors aligné avec l'arête du nez de l'utilisateur. Le nez sert alors de séparateur (ou de déflecteur) permettant de séparer l'aérosol projeté par le nébuliseur puis de le diriger, par gravité, vers la partie buccale du corps de masque en passant le long du visage, de part et d'autre du nez de l'utilisateur. Ainsi, l'aérosol ne passant pas devant le nez de l'utilisateur, il n'est pas entraîné par l'air expiré par l'utilisateur et n'est pas perdu ni ne contamine l'air extérieur.

La deuxième ouverture comprend un tube (108), aussi appelé tube d'inhalation, débouchant dans la partie buccale (ou partie basse) du corps de masque (102). Le tube (108) possède, par définition, deux extrémités. Le tube peut être seulement un trou dans le corps de masque, la longueur du tube étant l'épaisseur du corps de masque. Dans un cas particulier, le trou peut aussi être composé d'un ensemble de trous réunis dans une aire relativement restreinte de la partie buccale du corps de masque (102). Cet ensemble de trous peut alors être considéré comme le tube (108). L'extrémité du tube (108) débouchant dans le corps de masque (102) forme, avec ce dernier, une surface d'intersection qui est caractérisée par une direction normale (N2). La direction normale (N2) forme un angle (α) supérieur ou égale à 0° et inférieur ou égal à 90°, de préférence entre 10° et 75°, avec le plan moyen (S). Cette ouverture permet notamment le passage de l'air extérieur vers l'intérieur du masque lors de l'inspiration par l'utilisateur. La position de la surface d'intersection sur le corps de masque (102) dans la partie buccale du corps de masque (102) comme décrite ci-dessus est avantageuse car, par gravité, l'aérosol qui est entré par la première ouverture s'accumule dans la partie buccale du masque. L'air inspiré par l'utilisateur entre alors par la deuxième ouverture (108) débouchant elle aussi dans la partie buccale du masque. Ainsi, une grande proportion de l'aérosol qui s'est accumulé dans cette partie inférieure (buccale) du masque sera inspiré et pourra être déposée dans les voies respiratoires. De manière avantageuse, l'axe longitudinal du tube (108) peut être compris dans le plan vertical du corps de masque (102). Ceci est avantageux car le tube d'inhalation (108) est alors aligné avec les narines et la bouche de l'utilisateur permettant ainsi un mouvement plus naturel de l'air respiré par l'utilisateur.

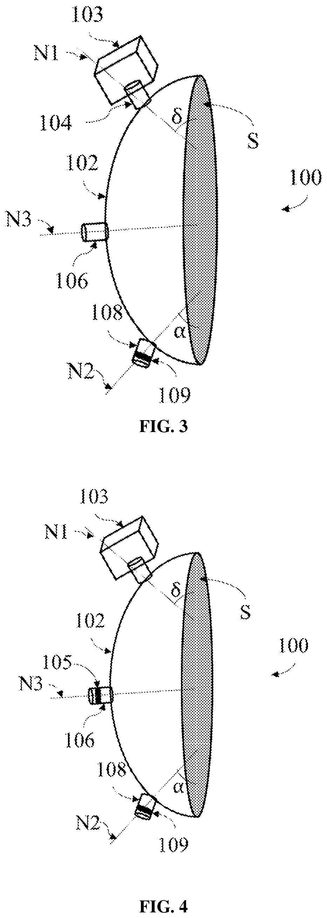

Dans un mode de réalisation montré à la figure 3, le masque facial comprend en outre une troisième ouverture. Cette ouverture comprend un tube (106), aussi appelé tube d'expiration, débouchant dans la partie centrale du corps de masque (102). Le tube (106) possède, par définition, deux extrémités. Le tube peut être seulement un trou dans le corps de masque, la longueur du tube étant l'épaisseur du corps de masque. L'extrémité débouchant dans le corps de masque (102) forme, avec ce dernier, une surface d'intersection qui est caractérisée par une direction normale (N3). La direction normale (N3) est sensiblement perpendiculaire avec le plan moyen (S). De manière avantageuse, l'axe longitudinal du tube d'expiration (106) peut être compris dans le plan vertical du corps de masque (102). Ceci est avantageux car le tube (106) est alors aligné simultanément avec les narines et la bouche de l'utilisateur permettant ainsi un mouvement plus naturel de l'air expiré par l'utilisateur.

Lorsque le masque facial comprend la troisième ouverture, la seconde extrémité du tube d'inhalation (108) est connectée à une valve unidirectionnelle (109) permettant une communication uniquement de l'extérieur vers l'intérieur du corps de masque (102). Ceci est avantageux car la valve unidirectionnelle (109) empêche l'air expiré de sortir par la deuxième ouverture. Ainsi, l'air expiré sort uniquement de la troisième ouverture comprenant le tube d'expiration (106). L'aérosol accumulé dans la partie buccale du masque n'est donc pas expulsé vers l'extérieur pendant l'expiration.

Dans un mode de réalisation avantageux représenté à la figure 4, la seconde extrémité du tube d'expiration (106) est connectée à une valve unidirectionnelle (105) permettant une communication de l'intérieur du corps de masque (102) vers l'extérieur. Ceci est avantageux car l'air inspiré par l'utilisateur passe alors uniquement par le tube d'inhalation (108). Ainsi, cela permet d'inhaler une plus grande proportion d'aérosol car ce dernier, comme expliqué plus haut, s'accumule, par gravité dans la partie buccale du masque, là où est positionné la deuxième ouverture comprenant le tube d'inhalation (108).

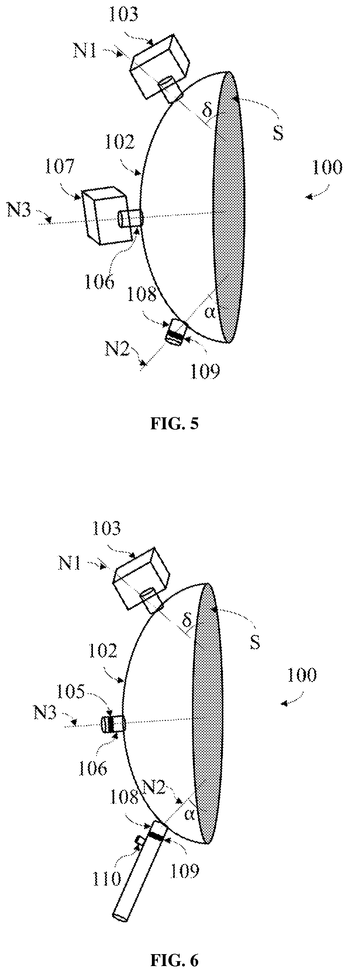

Dans un mode de réalisation avantageux représenté à la figure 5, la seconde extrémité du tube d'expiration tube (106) est connectée à un filtre (107). Dans un mode de réalisation spécifique, le filtre (107) est un filtre expiratoire retenant les fines gouttelettes lors de l'expiration. Ceci est avantageux car le filtre permet de protéger l'air extérieur d'une contamination d'aérosol durant l'expiration.

La valve unidirectionnelle (105) et le filtre (107) peuvent être connectés séparément ou simultanément au tube d'expiration (106). Dans un mode de réalisation, seule la valve unidirectionnelle (105) est connectée au tube d'expiration (106). Dans un mode de réalisation alternatif, seul le filtre (107) est connecté au tube d'expiration (106). Dans un autre mode de réalisation, la valve unidirectionnelle (105) et le filtre (107) sont connectés au tube d'expiration (106). Dans ce mode de réalisation, le filtre (107) peut être positionné en amont de la valve unidirectionnelle (105), c'est-à-dire entre la valve unidirectionnelle (105) et l'intérieur du corps de masque (102). Ceci permet de protéger la valve (105) d'une contamination par les gouttelettes d'aérosol et d'un dysfonctionnement potentiel suite à son colmatage.

Dans le mode de réalisation dans lequel le tube d'inhalation (108) comprend une valve unidirectionnelle (109), le tube d'inhalation (108) peut comprendre en outre une entrée (110), représentée à la figure 6, destinée à être reliée à une source de gaz sec telle qu'un réservoir d'air sec ou d'oxygène sec. La source de gaz sec peut être une bouteille sous pression reliée directement au masque facial via, par exemple, un tube annelé connecté à l'entrée (110). Dans un mode de réalisation alternatif, par exemple en milieu hospitalier, la source de gaz sec est un réservoir central. L'entrée (110) du masque facial est alors reliée via, par exemple, un tube annelé et un détendeur mural à une des sorties d'air du réservoir central. L'utilisation d'air sec ou d'oxygène sec est avantageuse car elle permet un rinçage plus efficace du masque pendant l'inspiration. En effet, le gaz sec limite la condensation de vapeur d'eau sur les gouttelettes d'aérosol pour éviter le dépôt sur la paroi interne du corps de masque (102). Cela permet donc de réduire la perte d'aérosol et d'augmenter l'efficacité du dépôt de l'aérosol dans les voies respiratoires de l'utilisateur. L'addition de gaz sec permet également l'évaporation des gouttelettes et donc une meilleure pénétration pulmonaire.

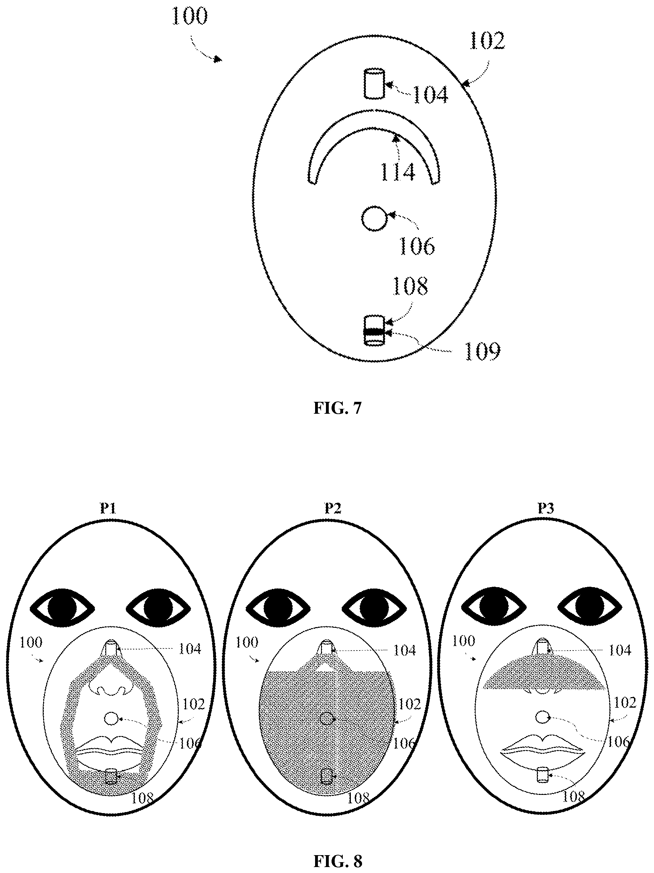

Dans un mode de réalisation représenté à la figure 7, le corps de masque (102) comprend en outre un répartiteur (114) (ou déflecteur) permettant de diriger de manière plus efficace l'aérosol de part et d'autre du nez de l'utilisateur et de limiter le dépôt de l'aérosol sur le nez de l'utilisateur. Le répartiteur (114) peut être une pièce rigide ou semi-rigide. Le répartiteur (114) peut se fixer de manière réversible (permettant de l'accrocher et de le décrocher de manière répétitive) à l'intérieur du corps de masque (102). De manière alternative, le répartiteur (114) peut être intégré à l'intérieur du corps de masque (102) de manière définitive durant la fabrication de ce dernier. Dans un mode de réalisation spécifique, le répartiteur (114) possède une forme arquée. Dans un mode de réalisation spécifique, le répartiteur (114) est positionné entre la première ouverture comprenant le tube de nébulisation (104) et la troisième ouverture comprenant le tube d'expiration (106). Dans un mode de réalisation plus spécifique, le répartiteur de forme arquée est fixé de manière à ce que le milieu de l'arc soit positionné entre la première ouverture comprenant le tube de nébulisation (104) et la pointe du nez de l'utilisateur. Ceci est avantageux car cela permet de répartir de manière plus uniforme l'aérosol de part et d'autre du nez. Dans un mode de réalisation encore plus spécifique, le répartiteur de forme arquée est prolongé de part et d'autre du nez de l'utilisateur pour créer deux canaux de passage étanches entre le nez et les parois latérales du masque. Ceci est avantageux car cela permet d'orienter la trajectoire de l'aérosol vers la partie basse du masque et d'augmenter l'efficacité de direction de l'aérosol de part et d'autre du nez. L'aérosol est alors dirigé en dehors de la trajectoire de l'air expiré par l'utilisateur ce qui diminue la quantité d'aérosol éjecté dans l'air extérieur. Cela contribue à la diminution de la contamination de l'air extérieur et à l'augmentation de l'efficacité de dépôt dans les voies respiratoires.

De manière avantageuse, les tubes (104, 106, 108) définissent de petits volumes. Ceci est avantageux car cela permet de réduire le volume du masque. Le volume du masque est défini comme la somme du volume défini par le corps de masque (102) et le plan moyen (S) et du volume de chacun des tubes (104, 106, 108). Un faible volume de masque permet, lorsque des valves unidirectionnelles sont présentes dans le masque facial, le déclenchement plus rapide de l'ouverture de ces valves par réduction du volume de détente du gaz. Par exemple, le volume du masque peut être réduit en diminuant la longueur des tubes (104, 106, 108). Ainsi, la dimension longitudinale des tubes (104, 106, 108) de chacune des ouvertures peut être inférieure à 2 centimètres, de préférence inférieure à 1 centimètre. Dans un mode de réalisation spécifique, le volume du masque est sensiblement égal ou inférieur à la capacité pulmonaire d'un utilisateur moyen. Par exemple, pour un utilisateur adulte, le volume du masque peut être inférieur à 500 millilitres tandis que pour un enfant, le volume du masque peut être inférieur à 100 millilitres, de préférence proche de 50 millilitres.

Par exemple, le masque et les tubes sont fabriqués en une matière de type polypropylène PP, polyéthylène PE ou polyéther-éthercétone PEEK. Le masque et les tubes sont préférentiellement sans phtalate ni PVC. Le masque et les tubes sont préférentiellement réalisés en une matière non susceptible de se charger électrostatiquement.

La présente divulgation concerne aussi une utilisation du système (100) décrit ci-dessus pour administrer un produit dans les voies respiratoires d'un utilisateur.

La première étape d'utilisation est d'introduire un principe actif en liquide dans le nébuliseur à tamis (103). Le principe actif peut être un médicament utilisé dans un traitement thérapeutique. Le principe actif peut être un pathogène ou un marqueur, utilisé dans le cadre de tests cliniques ou de recherche. Dans ce cas, limiter au maximum la dispersion du pathogène hors du masque est important, et une administration efficace - quantitative - du pathogène permet d'utiliser des quantités plus faibles de produits dangereux.

L'introduction du principe actif peut se faire par dissolution dans le cas d'un principe actif liquide ou par dispersion dans le cas d'un principe actif non-soluble.

Le système (100) est ensuite installé sur le nez et la bouche de l'utilisateur. Ceci est avantageux car cela permet une administration simultanée des voies respiratoires supérieures et inférieures, l'utilisateur pouvant inhaler à la fois par le nez et la bouche. Dans un mode de réalisation spécifique, afin d'augmenter l'efficacité de l'utilisation du système, l'utilisateur est installé en position assis, debout ou semi-assis/semis-couché.

Le nébuliseur à tamis (103) génère en continu un aérosol comprenant le principe actif introduit. Un autre avantage à l'utilisation du nébuliseur à tamis est qu'il permet d'adapter le débit d'aérosol et la taille des gouttelettes à l'utilisateur. Ainsi, dans un mode de réalisation spécifique, le débit du nébuliseur est compris entre 0,01 et 1 millilitre/minute et/ou la taille des gouttelettes d'aérosol produites est comprise entre 0,1 et 10 microns. De préférence, le débit d'aérosol est compris entre 0,05 millilitres/minute et 0,5 millilitres/minute, de manière optimale entre 0,05 et 0,1 millilitre/minute. Par ailleurs et indépendamment du débit du nébuliseur, la taille des gouttelettes d'aérosol produites est de préférence comprise entre 0,5 et 5 microns, de manière optimale entre 2 et 5 microns. Ceci est avantageux car l'utilisation de ces gammes de débit permet de limiter la perte d'aérosol par condensation des gouttelettes sur la paroi interne du masque. De plus, ces gammes de taille de gouttelettes est assez faible pour permettre une meilleure efficacité de dépôt dans les voies respiratoires inférieures.

La génération en continu de l'aérosol permet une utilisation du système (100) de la présente invention de manière la plus efficace possible. En effet, un cycle de respiration est divisé en trois phases représentées à la figure 8 : l'expiration (P1), la pause respiratoire (P2) et l'inspiration (P3).

Durant l'expiration (P1), l'air contenu dans les voies respiratoires de l'utilisateur est évacué vers l'extérieur du corps de masque (102). Dans le mode de réalisation de la figure 8, le corps de masque comprend trois tubes (104, 106 et 108). Le tube d'inspiration (108) comprend donc une valve unidirectionnelle (non représentée) permettant le passage de l'extérieur à l'intérieur du masque. L'air expiré ne peut donc sortir du corps de masque (102) que par le tube d'expiration (106).

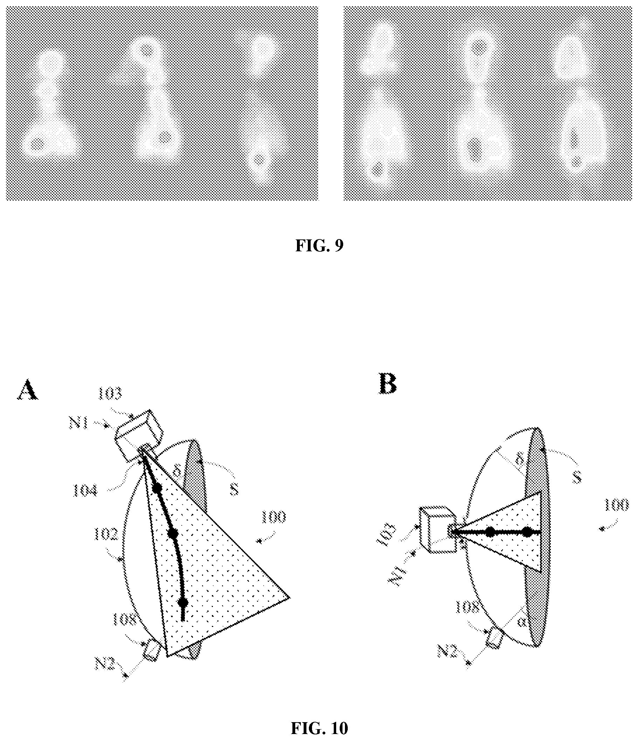

Pendant la pause respiratoire (P2), l'aérosol (représenté par un nuage de points dans la figure 8) s'accumule dans le masque grâce à sa production en continu par le nébuliseur à tamis (103). Avantageusement, pour un utilisateur debout, assis ou semi-assis/semi-couché, le tube de nébulisation (104) étant positionné au-dessus de l'arête du nez, l'aérosol produit par le nébuliseur à tamis (103) est principalement dirigé de part et d'autre du nez de l'utilisateur, de préférence substantiellement parallèlement au visage de l'utilisateur, et tombe, par gravité, dans la partie buccale du masque (figure 10A). Un volume de masque peu important permet un remplissage plus rapide du masque.

Dans les masques faciaux connus, l'aérosol est envoyé dans le masque via une ouverture située dans la partie centrale du corps de masque et de manière perpendiculaire au plan moyen (S) (figure 10B), le flux d'aérosol n'est donc pas dirigé de part et d'autre de l'arête du nez vers la partie buccale du corps de masque. Ainsi, aucune accumulation dans la partie buccale du masque n'est possible car le flux d'aérosol est dirigé perpendiculairement à la force de gravité. Durant le temps de la pause inspiratoire (P2), le flux d'aérosol n'a donc pas le temps de changer de direction et de se diriger vers la partie buccale du masque. Ceci a pour conséquence de réduire l'efficacité de dépôt dans les voies respiratoires.

Lors de l'inspiration (P3), l'air extérieur est inspiré par le tube d'inspiration (108). Dans le mode de réalisation où le tube d'expiration (106) ne comprend pas de valve unidirectionnelle, l'air peut aussi être inspiré par le tube d'expiration (106). Le masque est alors rincé par l'air inspiré et les gouttelettes d'aérosol sont alors inhalées pour être déposées dans les voies respiratoires de l'utilisateur.

Un résultat similaire est obtenu lorsque le système (100) est utilisé sur une personne couchée.

### EXEMPLES

La présente invention est aussi illustrée par les exemples suivants. Le matériel utilisé et les résultats des exemples 1-3 sont résumés dans le Tableau 1.

### [Table 1]

**Tableau 1 : Résultats expérimentaux**

| | Exemple 1 | | Exemple 2 | | | Exemple 3 | | |
|---|---|---|---|---|---|---|---|---|
| | INV | AA | INV | AA | AA | INV | | INV |
| Condition | In vivo | In vivo | In vitro | In vitro | In vivo | In vitro | In vitro | In vivo |
| Masque | Fig. 3 | Fig. 3 | Fig. 3 | AA | AA | Fig. 3 | Fig. 3 | Fig. 3 |
| Nébuliseur | Tamis statique | Micro Cirrus^{™} | Tamis statique | Tamis statique | Tamis statique | Tamis statique | Tamis statique | Tamis vibrant |
| Débit (mL/min) | 0,8 | 0,7 | 0,5 | 07 | 0,7 | 1 | 0,2 | 0,08 |
| Taille des gouttelettes (µm) | 4 | 4 | 4,2 | 4,2 | 4,2 | 3,2 | 3,2 | 3,2 |
| Rendement de nèbulisation (%) | 36 | 2,8 | 27 | 9 | 16 | 29 | 52 | 41 |
| Rendement pulmonaire (%) | 12,5 | 0,5 | 24 | 7 | 6 | 25 | 43 | 17 |

### Example 1 : Comparaison des rendements de nébulisation

Dans cet exemple, le rendement de nébulisation est comparé, après une séance de nébulisation de 10 minutes, entre un système d'inhalation selon le mode de réalisation de la figure 3 de l'invention (INV) - c'est-à-dire comprenant un nébuliseur à tamis et un masque facial comprenant trois ouvertures - et un système comprenant le même masque facial et un nébuliseur pneumatique commercial de type Micro CirrusTM (AA). Le nébuliseur à tamis comprend une membrane statique produisant un aérosol radioactif de 4 microns MMAD à un débit de 0.8 mL/minute. Le nébuliseur pneumatique fonctionne à l'aide d'une source d'air comprimé introduit avec un débit de 8 litres/minute et produit un aérosol radioactif de 4 microns MMAD avec un débit de 0.7 mL/minute. Ces aérosols radioactifs ont été administrés à trois primates non humain de type macaque.

Les résultats ont montré un rendement de nébulisation dans les voies respiratoires des macaques de 2.8% avec le nébuliseur pneumatique (AA) et de 36% avec le nébuliseur à tamis (INV) selon l'invention. Le rendement pulmonaire est de 0.5% avec le nébuliseur pneumatique (AA) et de 12.5% avec le nébuliseur à tamis (INV).

L'utilisation d'un nébuliseur à tamis permet donc bien d'augmenter le rendement de nébulisation et le dépôt dans les poumons par rapport à un nébuliseur pneumatique. Le système selon l'invention comprenant un nébuliseur à tamis permet donc bien d'augmenter l'efficacité de nébulisation.

### Example 2 : Comparaison avec les masques faciaux connus

Dans cet exemple, le taux de dépôt est comparé après une séance de nébulisation de 10 minutes avec un nébuliseur à tamis produisant un aérosol de 4,2 microns MMAD branché successivement sur deux masques faciaux différents. Le premier masque, connu de l'art antérieur (AA), est un mode de réalisation du masque décrit dans la demande de brevet français FR 2985909. Il est équipé de valves unidirectionnelles et d'une chambre de stockage connectée à l'une des valves unidirectionnelles d'une part et au corps de masque d'autre part. L'aérosol est produit dans la zone de stockage. Durant la phase expiratoire l'utilisateur expire l'air au travers de la valve unidirectionnelle non-connectée à la zone de stockage. Lors de l'inspiration, l'aérosol accumulé dans la zone de stockage est inhalé par l'utilisateur à l'aide de l'air mobilisé via la deuxième valve unidirectionnelle. Avec ce masque, le débit du nébuliseur à tamis est de 0,7 mL/minute. Le deuxième masque facial (INV) est le masque facial selon le mode de réalisation représenté à la figure 3 comprenant trois ouvertures et un nébuliseur à tamis produisant un aérosol avec un débit de 0,5 mL/minute.

Les aérosols ont été administrés à un modèle anatomique in vitro de singe Vervet connecté à une pompe respiratoire simulant la respiration de l'animal (25 mL, 30 respirations/minute, volume d'air inspiré (I) égal au volume d'air expiré € - I/E=1).

Les résultats in vitro ont montré un rendement de nébulisation dans les voies respiratoires de 9% avec le premier système (AA) et de 27% avec le deuxième système (INV). Le rendement pulmonaire est de 7% avec le premier système (AA) et de 24% avec le deuxième système (INV).

L'aérosol a aussi été administré à trois primates non humain de type macaque (in vivo) avec le masque connu de l'art antérieur (AA) et avec un débit de nébulisation de 0,7 mL/minute.

La distribution du dépôt d'aérosol dans les voies respiratoires inférieures et supérieures des trois macaques (in vivo, mesure par scintigraphie) est représentée sur la partie gauche de la figure 9. Sur l'image en niveaux de gris, plus le dépôt est grand, plus la zone est sombre. Les résultats in vivo ont montré un rendement dans les voies respiratoires de 16% et un rendement pulmonaire de 6%.

Le système selon l'invention permet donc bien d'augmenter le rendement de nébulisation et le dépôt dans les poumons par rapport à un nébuliseur pneumatique comparé à un système comprenant un masque facial connu de l'art antérieur.

### Example 3 : Efficacité en fonction du débit

Dans cet exemple, une première comparaison du rendement de nébulisation in vitro est effectuée après deux séances de nébulisation utilisant le système selon le mode de réalisation de la figure 3 (INV) - c'est-à-dire, comprenant trois ouvertures et un nébuliseur à tamis. A chaque séance de nébulisation, la taille de gouttelettes produites par le nébuliseur est identique tandis que le débit de nébulisation diffère. Ainsi, lors de la première séance, le nébuliseur à tamis produit un aérosol de 3,2 microns à un débit de 1 mL/minute tandis que lors de la deuxième séance, le nébuliseur à tamis produit un aérosol de 3,2 microns à un débit - plus faible - de 0,2 mL/minute. Les aérosols ont été administrés à un modèle anatomique in vitro de singe Vervet connecté à une pompe respiratoire simulant la respiration de l'animal (25 mL, 30 respirations/minute, I/E=1).

Les résultats in vitro ont montré un rendement de nébulisation dans les voies respiratoires de 29% avec le débit le plus important et de 52% avec le débit le plus faible. Le rendement pulmonaire était de 25% avec le débit le plus important et de 43% avec le débit le plus faible.

Le rendement de nébulisation a aussi été mesuré in vivo après une séance de nébulisation utilisant le système selon le mode de réalisation de la figure 3 (INV) - c'est-à-dire, comprenant trois ouvertures et un nébuliseur à tamis. Le nébuliseur à tamis produit un aérosol radioactif de 3,2 microns à un débit - encore plus faible - de 0,08 mL/minute. L'aérosol a été administré à trois primates non humain de type macaque.

La distribution du dépôt d'aérosol dans les voies respiratoires inférieures et supérieures des trois macaques (in vivo, mesure par scintigraphie) est représentée sur la partie droite de la figure 9. Sur l'image en niveaux de gris, plus le dépôt est grand, plus la zone est sombre. Les résultats in vivo ont montré un rendement dans les voies respiratoires de 41% et un rendement pulmonaire de 17%.

Cet exemple montre donc bien qu'un débit plus faible permet un rendement de nébulisation plus élevé. L'invention étant utilisée avec un débit faible (entre 0,01 et 1 mL/minute, de préférence entre 0,05 mL/minute et 0,5 mL/minute), cela permet un plus grand rendement de nébulisation et pulmonaire.

### Example 4 : Comparaison des rendements de nébulisation en fonction de la position de la première ouverture

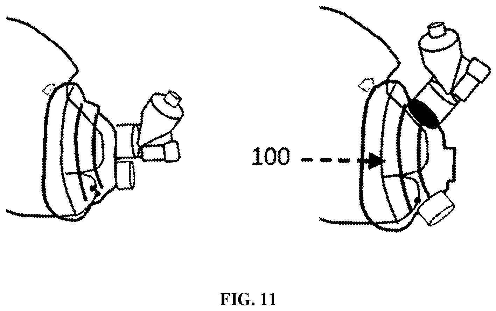

Dans cet exemple, le rendement de nébulisation est comparé, après une séance de nébulisation de 10 minutes, entre un système d'inhalation selon le mode de réalisation de la figure 3 de l'invention - c'est-à-dire comprenant un nébuliseur à tamis et un masque facial comprenant trois ouvertures - représenté sur la droite de la figure 11 et un système comprenant le même nébuliseur mais monté sur un masque facial comprenant seulement deux ouvertures et dont l'ouverture reliée au nébuliseur est positionnée dans la partie centrale du corps de masque comme représenté sur la gauche de la figure 11. Ce dernier système envoie donc l'aérosol perpendiculairement à la face du sujet.

Le nébuliseur à tamis comprend une membrane statique produisant un aérosol radioactif de 4 microns MMAD à un débit de 0.8 mL/minute. Cet aérosol radioactif a été administré à trois primates non humain de type macaque.

Les rendements de nébulisation pour chacun des deux masques sont mesurés. Les résultats obtenus montrent que le masque facial comprenant seulement deux ouvertures ne permet de délivrer que 12% de la dose dans les voies respiratoires alors que le masque de droite, selon l'invention, permet de délivrer 54% de la dose. L'emplacement de la première ouverture et la distanciation entre la première et la deuxième ouverture permettent donc d'améliorer significativement les performances du rendement de nébulisation.

## Revendications

1. Un système (100) d'inhalation comprenant un nébuliseur à tamis (103) et un masque facial comprenant :
- Un corps de masque (102) configuré pour couvrir le nez et la bouche d'un utilisateur lorsque ledit corps de masque (102) est placé sur l'utilisateur et délimité par un bord de masque définissant une surface **caractérisée par** un plan moyen (S),
- Une première ouverture comprenant un tube (104) débouchant dans la partie nasale du corps de masque (102) et comprenant une première extrémité dont l'intersection avec le corps de masque (102) définit une surface dont la direction normale (N1) forme un angle (δ) supérieur à 10° et inférieur ou égal à 90°, de préférence inférieur ou égal à 75°, avec le plan moyen (S) et une deuxième extrémité connectée au nébuliseur à tamis (103), la partie nasale du corps de masque (102) étant la partie du corps de masque (102) située, lorsque le masque est porté par un utilisateur assis ou debout, au-dessus de la pointe du nez, et
- Une deuxième ouverture comprenant un tube (108) débouchant dans la partie buccale du corps de masque (102) et comprenant une première extrémité dont l'intersection avec le corps de masque (102) définit une surface dont la direction normale (N2) forme un angle (α) supérieur à 0° et inférieur à ou égal à 90° avec le plan moyen (S), la partie buccale du corps de masque (102) étant la partie du corps de masque (102) située, lorsque le masque est porté par un utilisateur assis ou debout, en dessous de la lèvre inférieure.

2. Le système (100) selon la revendication **1,** dans lequel la deuxième ouverture comprenant un tube (108) comprend une seconde extrémité connectée à une valve unidirectionnelle (109) permettant une communication de l'extérieur vers l'intérieur du corps de masque (102), le masque facial comprenant en outre une troisième ouverture comprenant un tube (106) débouchant dans la partie centrale du corps de masque (102) et comprenant une première extrémité dont l'intersection avec le corps de masque (102) définit une surface dont la direction normale (N3) est sensiblement perpendiculaire au plan moyen (S).

3. Le système (100) selon la revendication **2,** dans lequel la troisième ouverture comprenant un tube (106) comprend une deuxième extrémité connectée à une valve unidirectionnelle (105) permettant une communication de l'intérieur du corps de masque (102) vers l'extérieur.

4. Le système (100) selon la revendication **2** ou **3,** dans lequel la dimension longitudinale des tubes de chacune des ouvertures (104, 106, 108) est inférieure à 2 centimètres, de préférence inférieure à 1 centimètre.

5. Le système (100) selon l'une des revendications **2** à **4,** dans lequel la troisième ouverture (106) comprend une deuxième extrémité connectée à un filtre (107).

6. Le système (100) selon l'une des revendications **2** à **5,** dans lequel la deuxième ouverture (108) comprend en outre une entrée (110) destinée à être reliée à une source de gaz sec, la valve unidirectionnelle (109) de la deuxième ouverture (108) étant située entre ladite entrée (110) et le corps de masque (102).

7. Le système (100) selon l'une des revendications **2** à **6,** dans lequel le corps de masque (102) comprend en outre un répartiteur (114) permettant de diriger un aérosol produit par le nébuliseur à tamis (103) de part et d'autre du nez de l'utilisateur lorsque le masque facial est placé sur l'utilisateur, ledit répartiteur (114) étant positionné entre la première ouverture (104) et la troisième ouverture (106).

8. Le système (100) selon l'une des revendications **1** à **7,** dans lequel le corps de masque (102) possède un volume inférieur à 500 millilitres.

9. Le système (100) selon l'une des revendications **1** à **8,** dans lequel le bord du corps de masque (102) est fabriqué dans un matériau souple.

## Patentansprüche

1. Inhalationssystem (100), umfassend einen Siebvernebler (103) und eine Gesichtsmaske, umfassend:
- einen Maskenkörper (102), der so eingerichtet ist, dass er die Nase und den Mund eines Benutzers abdeckt, wenn der Maskenkörper (102) auf dem Benutzer aufgesetzt wird, und der durch einen Maskenrand begrenzt ist, der eine Oberfläche definiert, die durch eine Mittelebene (S) gekennzeichnet ist,
- eine erste Öffnung, die ein Rohr (104) umfasst, das in den Nasenteil des Maskenkörpers (102) mündet und ein erstes Ende, dessen Schnittpunkt mit dem Maskenkörper (102) eine Oberfläche definiert, deren Normalenrichtung (N1) einen Winkel (δ) größer als 10° und kleiner oder gleich 90°, vorzugsweise kleiner oder gleich 75°, mit der Mittelebene (S) bildet, und ein zweites Ende umfasst, das mit dem Siebvernebler (103) verbunden ist, wobei der Nasenteil des Maskenkörpers (102) der Teil des Maskenkörpers (102) ist, der sich über der Nasenspitze befindet, wenn die Maske von einem sitzenden oder stehenden Benutzer getragen wird, und
- eine zweite Öffnung, die ein Rohr (108) umfasst, das in den Mundteil des Maskenkörpers (102) mündet und ein erstes Ende umfasst, dessen Schnittpunkt mit dem Maskenkörper (102) eine Oberfläche definiert, deren Normalenrichtung (N2) einen Winkel (α) größer als 0° und kleiner oder gleich 90° mit der Mittelebene (S) bildet, wobei der Mundteil des Maskenkörpers (102) der Teil des Maskenkörpers (102) ist, der sich unterhalb der Unterlippe befindet, wenn die Maske von einem sitzenden oder stehenden Benutzer getragen wird.

2. System (100) nach Anspruch **1,** wobei die zweite Öffnung ein Rohr (108) umfasst, das ein zweites Ende umfasst, das mit einem Einwegventil (109) verbunden ist, das eine Strömungsverbindung von der Außenseite zur Innenseite des Maskenkörpers (102) hin ermöglicht, wobei die Gesichtsmaske ferner eine dritte Öffnung umfasst, die ein Rohr (106) umfasst, das in den Mittelteil des Maskenkörpers (102) mündet und ein erstes Ende umfasst, dessen Schnittpunkt mit dem Maskenkörper (102) eine Oberfläche definiert, deren Normalenrichtung (N3) im Wesentlichen senkrecht zur Mittelebene (S) liegt.

3. System (100) nach Anspruch **2,** wobei die dritte Öffnung, die ein Rohr (106) umfasst, ein zweites Ende umfasst, das mit einem Einwegventil (105) verbunden ist, das eine Strömungsverbindung von der Innenseite des Maskenkörpers (102) zur Außenseite hin ermöglicht.

4. System (100) nach Anspruch **2** oder **3,** wobei das Längsmaß der Rohre jeder der Öffnungen (104, 106, 108) weniger als 2 Zentimeter, vorzugsweise weniger als 1 Zentimeter beträgt.

5. System (100) nach einem der Ansprüche **2** bis **4,** wobei die dritte Öffnung (106) ein zweites Ende umfasst, das mit einem Filter (107) verbunden ist.

6. System (100) nach einem der Ansprüche **2** bis **5,** wobei die zweite Öffnung (108) ferner einen Eingang (110) umfasst, der dazu bestimmt ist, mit einer Trockengasquelle verbunden zu werden, wobei das Einwegventil (109) der zweiten Öffnung (108) zwischen dem Eingang (110) und dem Maskenkörper (102) angeordnet ist.

7. System (100) nach einem der Ansprüche **2** bis **6,** wobei der Maskenkörper (102) ferner einen Deflektor (114) umfasst, der es ermöglicht, ein von dem Siebvernebler (103) erzeugtes Aerosol auf beiden Seiten der Nase des Benutzers zu leiten, wenn die Gesichtsmaske auf dem Benutzer aufgesetzt wird, wobei der Deflektor (114) zwischen der ersten Öffnung (104) und der dritten Öffnung (106) positioniert ist.

8. System (100) nach einem der Ansprüche **1** bis **7,** wobei der Maskenkörper (102) ein Volumen von weniger als 500 Milliliter aufweist.

9. System (100) nach einem der Ansprüche **1** bis **8,** wobei der Rand des Maskenkörpers (102) aus einem weichen Material hergestellt ist.

## Claims

1. An inhalation system (100) comprising a mesh nebulizer (103) and a face mask comprising:
- a mask body (102) configured to cover the nose and the mouth of a user when said mask body (102) is placed on the user and bounded by a mask edge defining a surface **characterized by** a mean plane (S),
- a first opening comprising a tube (104) opening into the nasal portion of the mask body (102) and comprising a first end whose intersection with the mask body (102) defines a surface whose normal direction (N1) forms an angle (δ) greater than 10° and less than or equal to 90°, preferably less than or equal to 75°, with the mean plane (S), and a second end connected to the mesh nebulizer (103), the nasal portion of the mask body (102) being the part of the mask body (102) located, when the mask is worn by a seated or standing user, above the tip of the nose, and
- a second opening comprising a tube (108) opening into the buccal portion of the mask body (102) and comprising a first end whose intersection with the mask body (102) defines a surface whose normal direction (N2) forms an angle (α) greater than 0° and less than or equal to 90° with the mean plane (S), the buccal portion of the mask body (102) being the part of the mask body (102) located, when the mask is worn by a seated or standing user, below the lower lip.

2. The system (100) according to claim 1, wherein the second opening comprising a tube (108) comprises a second end connected to a one-way valve (109) allowing communication from the outside to the inside of the mask body (102), the face mask further comprising a third opening comprising a tube (106) opening into the central portion of the mask body (102) and comprising a first end whose intersection with the mask body (102) defines a surface whose normal direction (N3) is substantially perpendicular to the mean plane (S).

3. The system (100) according to claim 2, wherein the third opening comprising a tube (106) comprises a second end connected to a one-way valve (105) allowing communication from the inside of the mask body (102) to the outside.

4. The system (100) according to claim 2 or 3, wherein the longitudinal dimension of the tubes of each of the openings (104, 106, 108) is less than 2 centimeters, preferably less than 1 centimeter.

5. The system (100) according to any one of claims 2 to 4, wherein the third opening (106) comprises a second end connected to a filter (107).

6. The system (100) according to any one of claims 2 to 5, wherein the second opening (108) further comprises an inlet (110) intended to be connected to a dry gas source, the one-way valve (109) of the second opening (108) being located between said inlet (110) and the mask body (102).

7. The system (100) according to any one of claims 2 to 6, wherein the mask body (102) further comprises a deflector (114) allowing an aerosol produced by the mesh nebulizer (103) to be directed on either side of the user's nose when the face mask is placed on the user, said deflector (114) being positioned between the first opening (104) and the third opening (106).

8. The system (100) according to any one of claims 1 to 7, wherein the mask body (102) has a volume of less than 500 milliliters.

9. The system (100) according to any one of claims 1 to 8, wherein the edge of the mask body (102) is made of a flexible material.
